# EUROPEAN PATENT APPLICATION

(11) **EP 3 782 545 A1**
(43) Date of publication of application: **24.02.2021**
(21) Application number: 19192252.5
(22) Date of filing: 19.08.2019
(51) Int. Cl.: A61B 5/0428

(54) **ECG LEADSET**

(71) Applicant: Koninklijke Philips N.V., 5656 AG Eindhoven (NL)
(72) Inventor: VEENSTRA, Hugo, 5656 AE Eindhoven (NL); WEICHERT, Sven, 5656 AE Eindhoven (NL); LAMBERT, Nicolaas, 5656 AE Eindhoven (NL); PAULUSSEN, Igor Wilhelmus Franciscus, 5656 AE Eindhoven (NL)
(74) Representative: de Haan, Poul Erik

(57) **Abstract**

An ECG leadset has a plurality of ECG electrode connectors spaced along a connection bus. Each ECG electrode connector has a single connection region at which it connects to the connection bus. The single connection region may be considered to be a single location around the periphery of the ECG electrode connector. In this way each ECG electrode connector can be oriented such that the lead or leads which connect to it can be positioned to face away from the area of interest to a surgeon. Thus, the leads can be positioned to prevent a surgical procedure being hampered.

## Description

### FIELD OF THE INVENTION

This invention relates to ECG leadsets.

### BACKGROUND OF THE INVENTION

An ECG leadset comprises a set of leads which couple to ECG electrode connectors. The ECG electrode connectors are connected physically and electrically to patient electrodes (i.e. pads) which have been attached to a subject being monitored.

Within each lead, one or more wires lead from each ECG electrode connector to a plug terminal at which all of the ECG electrode connectors (and hence the patient electrodes) are connected to an ECG monitoring unit.

The ECG of a patient is routinely measured in clinical settings. During surgery it is common practice to monitor the ECG of a patient continuously.

ECG leadsets usually comprise a single and separate lead for each patient electrode, which extends from the plug terminal to the associated ECG electrode connector and hence patient electrode. Clinical leadsets typically interface with 3 to 10 patient electrodes. There are thus multiple leads per leadset. The likelihood of tangling of leads increases as the number of leads increases. It also means the time to connect the leadset to the patient electrodes increases. The leads may also hamper a surgical procedure if some of the leads run across areas where an operation must take place.

These problems have partly been addressed by the Draeger Monolead ECG leadset (trade mark). This connects all ECG electrode connectors via a common lead. Each ECG electrode connector has an input side for the common lead and an output side for the common lead, except the last one on the connection bus which has only an input side. One wire within the common lead terminates at that ECG electrode connector and makes electrical connection to a coupled patient electrode, and the other wires within the common lead pass that ECG electrode connector. Thus, each ECG electrode connector functions as a termination for one wire and as a bypass unit for all of the other wires.

This reduces the tangling problem and reduces the weight of the leadset, making it more comfortable for the patient when the leadset must be attached for a longer time.

The input side and output side are on diametrically opposed sides of the ECG electrode connector, so the ECG electrode connectors may be considered to be positioned along and on the common lead. However, it may still be difficult to position the common lead in a way which keeps clear the area for surgery, and the surgical procedure may still be hampered.

There is therefore a need for an improved ECG leadset.

### SUMMARY OF THE INVENTION

The invention is defined by the claims.

According to examples in accordance with an aspect of the invention, there is provided an ECG leadset, comprising:
a connection bus;
an electrical terminal at one end of the connection bus; and
a set of a plurality of ECG electrode connectors, each electrically connected to the connection bus at a different physical location along the bus, wherein each ECG electrode connector has a single connection region around the periphery of the ECG electrode connector at which it connects to the connection bus.

By connecting to the ECG electrode connectors at a single connection region, in particular on one side only, each ECG electrode connector can be oriented such that the lead or leads which extend from it can be directed away from the area of interest to a surgeon. Thus, the leads can be positioned to prevent a surgical procedure being hampered.

The design also enables a reduction in the total strain of the leadset to the patient, leading to better patient comfort.

The ECG electrode connectors may have any shape. Taken from the center of area of that shape, "a single connection region" may be considered to be a region which subtends less than 90 degrees at the center of area, or less than 60 degrees or less than 45 degrees. As will be clear from the description below, there may be only one lead which connects to the ECG electrode connector, however there may be an input lead and an output lead. They are next to each other, within this single region, and hence on the same side of the electrical connector. The term "a single region" should be understood accordingly.

By "connection bus" is meant a shared conductor bundle, which (at least at some locations along its length) has a collection of multiple "wires". The conductor bundle may then be manipulated and positioned as a single unit.

In a first set of examples, each ECG electrode connector of the plurality of electrode connectors, other than a last ECG electrode connector along the connection bus, connects to the connection bus through a spur connector which extends between the connection bus and the connection region.

In this way, the ECG electrode connectors are at the end of spurs which extend from the main connection bus. Only the wire within the connection bus, which is needed by the ECG electrode connector, is routed away from the connection bus down the spur. The spur may thus simply comprise one wire of the connection bus which has been separated from the bundle of wires to be routed to the ECG electrode connector. Thus, the connection bus can have one fewer wire in the bundle after the spur. Alternatively, the spur may comprise an additional wire which connects to an associated wire in the bus and the bus may have the same design along its length.

This avoids the need for special ECG electrode connectors that must themselves provide a bypass path for the other wires.

Each spur connector for example extends between the ECG electrode connector and a 3-terminal coupling along the connection bus. The 3-terminal coupling is a physical configuration rather than an electrical configuration, i.e. the wires which are for other ECG electrode connectors are not broken. Rather, one wire is physically tapped off from the connection bus to form the spur.

Each spur connector thus preferably comprises a single wire. This means the ECG electrode connector is easy and light to manipulate.

In a second set of examples, each ECG electrode connector of the plurality of electrode connectors connects to the connection bus with a connection bus input and a connection bus output, both at the connection region.

Thus, the ECG electrode connectors are physically along the connection bus (rather than spaced from the main part of the bus by spurs) with two connections to the connection bus. However, both connections to the bus are at the same general location, so they can both face away from the area of interest for surgery.

In all examples, there may be exactly 3, 4, 5 or 6 ECG electrode connectors along the connection bus. The desired number of electrode connectors depends on the type of ECG being performed.

The connection bus may be a first connection bus to which a first set of plurality of ECG electrode connectors connects, and the ECG leadset further comprises a second connection bus to which a second set of one or more ECG electrode connectors connects, wherein the electrical terminal is shared by the first and second connection buses.

In this way, there may be two connection buses extending from the terminal. This may make the routing to the full set of patient electrodes easier, for example using one bus for one side of the body and the other bus for the other side of the body.

There may be 5 ECG electrode connectors, three along the first connection bus and two along the second connection bus. This provides a 2-bus 5 lead leadset.

A coupling piece may be provided between the first and second connection buses and the shared electrical terminal, with a combined bus extending between the coupling piece and the shared electrical terminal. Thus, the two buses may be kept separate only for a required distance. They may be coupled together before they reach the shared electrical terminal.

The first connection bus may have a first end connector and the second connection bus may have a second end connector, the first and second end connectors being releasably couplable together to form the shared electrical terminal.

In this way, a leadset supports leadset extension, i.e. it may be used with a first number of ECG electrode connectors, or with a greater second number of ECG electrode connectors by coupling together multiple buses.

There may be a third connection bus to which a third set of one or more ECG electrode connectors connects, wherein the electrical terminal is shared by the first to third connection buses. Again, this may be used to facilitate coupling of the ECG electrode connectors with the patient electrodes.

There may be exactly 5 ECG electrode connectors, two along the first connection bus, two along the second connection bus and one along the third connection bus.

Thus, a connection bus may be for a single ECG electrode connector. However, in accordance with the invention, at least one connection bus is for a plurality of ECG electrode connectors.

When there are multiple connection buses each for a respective set of ECG electrode connectors, each ECG electrode connector of each set, other than a last ECG electrode connector along the respective connection bus, may connect to the respective connection bus through a spur connector. Thus, all buses use spur connections. Each spur connector then extends between the ECG electrode connector and a 3-terminal coupling along the respective connection bus.

Alternatively, again when there are multiple connection buses each for a respective set of ECG electrode connectors, each ECG electrode connector of each set may connect to the respective connection bus with a connection bus input and a connection bus output, both at the connection region. Thus, all buses use forked connections.

It is of course also possible to mix different types of connection bus within a single system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known 5-lead leadset, known as a monolead;
Figure 2 shows a first example of a leadset in accordance with the invention;
Figure 3 shows a second example of a leadset in accordance with the invention;
Figure 4 shows a third example of a leadset in accordance with the invention;
Figure 5 shows a fourth example of a leadset in accordance with the invention;
Figure 6 shows a fifth example of a leadset in accordance with the invention;
Figure 7 shows a sixth example of a leadset in accordance with the invention;
Figure 8 shows a seventh example of a leadset in accordance with the invention;
Figure 9 shows an eighth example of a leadset in accordance with the invention; and
Figure 10 shows an eighth example of a leadset in accordance with the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ECG leadset in which a plurality of ECG electrode connectors are spaced along a connection bus. Each ECG electrode connector has a single connection region at which it connects to the connection bus. The single connection region may be considered to be a single location around the periphery of the ECG electrode connector. In this way, each ECG electrode connector can be oriented such that the lead or leads which connect to it can be positioned to face away from the area of interest to a surgeon. Thus, the leads can be positioned to prevent a surgical procedure being hampered.

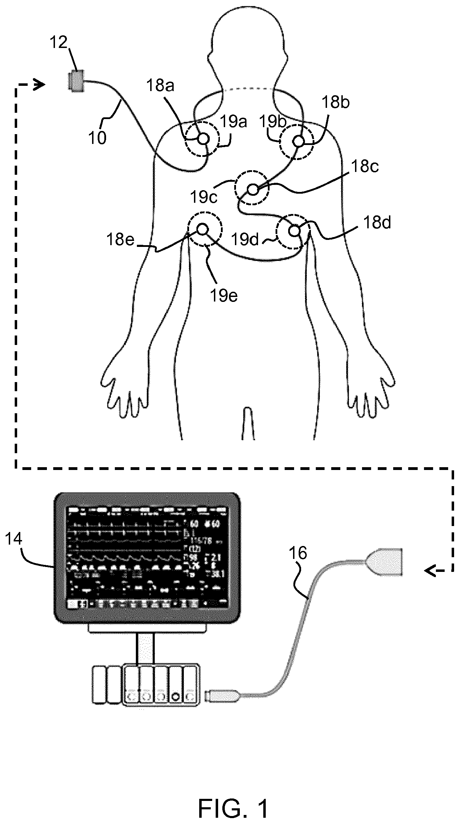

Figure 1 shows the a known 5-lead leadset, known as a "monolead".

The leadset comprises a connection bus 10 having an electrical terminal 12 at one end. This terminal 12 provides a connection between the leadset and an ECG monitoring unit 14, usually via a trunk cable 16 as shown in Figure 1. The trunk cable for example includes protection resistors.

A set of a plurality of ECG electrode connectors 18a to 18e is electrically connected to the connection bus 10 at a different location along the bus. The connection bus has at least one wire for each ECG electrode connector 18a to 18e, and the electrical terminal 12 has at least one connection terminal for each wire within the connection bus.

Inside the ECG electrode connectors 18a to 18e, a connection is made between the respective patient electrode 19a to 19e and the associated wire and an electrical bypass is provided for wires of the electrode connectors that are connected further downstream of the connection bus.

Thus, the number of wires in the connection bus tapers from 5 to 1 along its length.

Figure 1 shows one possible order of the ECG electrode connections and the resulting typical routing of a 5-lead monolead. It can be seen that the wiring near the ECG electrode connector 18c may interfere with the surgeon during heart surgery.

In the description above, the following terms are used. The leadset is the combination of the connection bus 10, terminal 12 and ECG electrode connectors 18a to 18e. The electrode connectors connect to the patient electrodes (i.e. the ECG electrodes, or ECG pads). The connection bus comprises a bundle of wires. The same terminology will be used throughout this document.

The electrodes are ordered "a to "e" in their positions along the connection bus. Connector 18a is the RA electrode, 18b is the LA connector, 18c is the center connector 18d is the LL connector and 18e is the RL connector. Even though the ordering along the bus or buses is different in the examples below, this naming convention is used in all of the examples below for 5 leadset connectors.

The leadset of the invention also uses a connection bus, but each electrode connector has a single connection region at which it connects to the connection bus (either directly or via a spur). This connection region is at one side of the ECG electrode connector, and this may be considered to be a single location. The aim is to enable the area where surgery must take place to be kept mostly free of leads, and to provides greater flexibility in the lead arrangement. The use of a connection bus enables a reduced number of leads compared to the standard design of a single lead for each ECG electrode connector, reducing the likelihood of tangling.

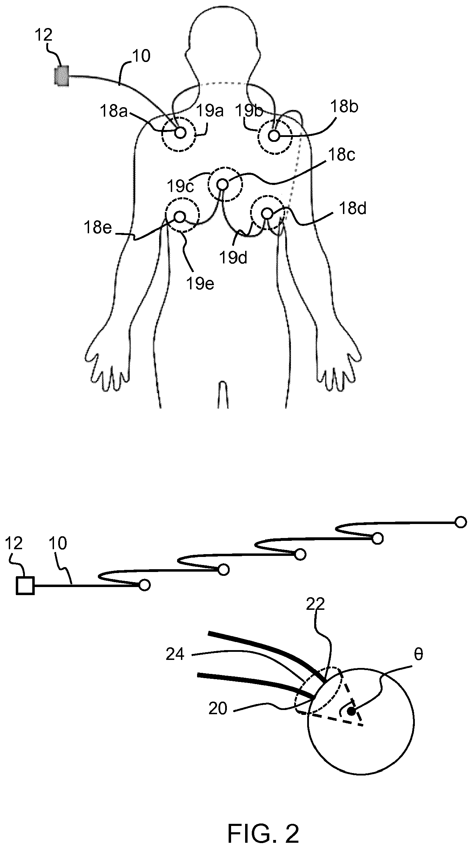

Figure 2 shows a first example in accordance with the invention. It shows a 5-lead leadset with 5 ECG electrode connectors 18a to 18e along a connection bus 10. The top image shows how the leadset may be applied to the body. The middle image shows the electrical connection between the ECG electrode connectors 18a to 18e. The bottom image shows one ECG electrode connector in enlarged form to show the connections to it.

Each ECG electrode connector connects to the connection bus with a connection bus input 20 and a connection bus output 22, both within a single connection region 24.

This general configuration will be described as a "forked" arrangement, as will be clear from the electrical connection diagram in Figure 2.

This connection region 24 may be considered to be at a single location, rather than at separate locations on opposite sides. A single region may be considered to be an area sufficiently small that it all fits within an angle θ of 90 degrees (or even within 60 or 45 degrees) to a center of area of the ECG electrode connector.

Thus, the ECG electrode connectors 18a to 18e are physically along the connection bus with two connections to the connection bus. However, both connections to the bus are at the same general location, so they can both face away from the area of interest for surgery. This can be seen in Figure 2.

The ECG electrode connectors provide a bypass for the wires which are not to connect to the patient electrode at that location.

This bypass does not need to involve a break in the wire. Rather, the wire is physically routed on from the ECG electrode connector, whereas the wire of interest is terminated so that electrical contact is made to the patient electrode.

Thus, the number of wires in the bus decreases along its length in the same way as mentioned above.

The order of the ECG electrode connectors along the connection bus is selected to achieve a desired cable routing when applied to the patient. As shown, the order is top right (of the patient's chest) =RA, top left =LA, bottom left =LL, center, bottom right =RL. Thus, the order along the bus is different to that shown in Figure 1, namely 18a, 18b, 18d, 18c, 18e.

By applying electrode connectors with the input 20 and output 22 placed on the same side, the connection bus is kept away from a large part of the chest area near the heart. Therefore, the leadset is less interfering to a surgical procedure.

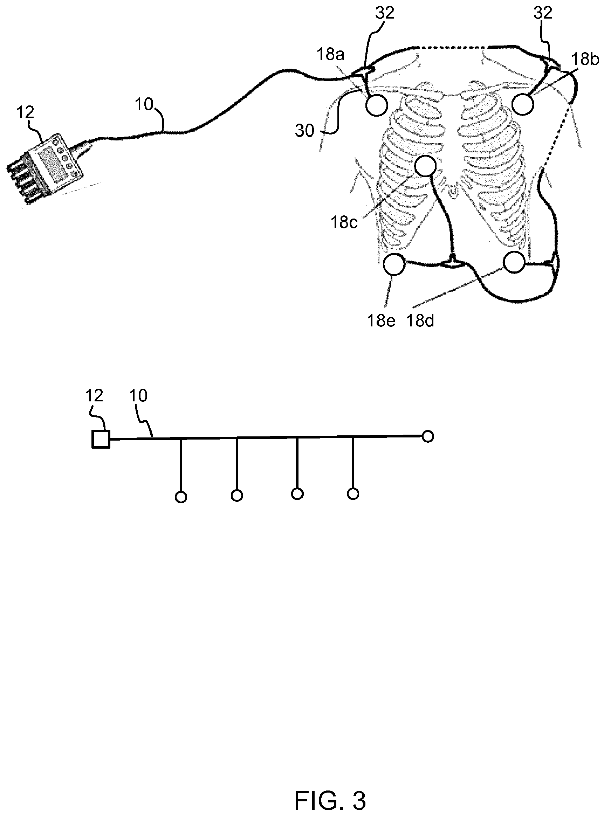

Note that the patient electrodes are not shown in Figures 3 to 9, to make the figures less cluttered.

Figure 3 shows a second example in accordance with the invention. It again shows a 5-lead leadset with 5 ECG electrode connectors 18a to 18e along a connection bus 10. The top image shows how the leadset may be applied to the body and the bottom image shows the electrical connection between the ECG electrode connectors 18a to 18e.

This general configuration will be described as a "spur" arrangement.

In this example, each ECG electrode connector 18a to 18e (other than a last ECG electrode connector), connects to the connection bus through a spur connector 30 which extends between the connection bus 10 and the connection region of the ECG electrode connector. In this case, the connection region includes only one lead connection.

The ECG electrode connectors are thus at the end of spurs which extend from the main connection bus 10. Only the wire within the connection bus, which is needed by the ECG electrode connector, is routed away from the connection bus down the spur. The spur may thus simply comprise one wire of the connection bus which has been separated from the bundle of wires, to be routed to the ECG electrode connector. Thus, the connection bus will have one fewer wire in the bundle after the spur.

This avoids the need for special ECG electrode connectors that must provide a bypass path for the other wires. The bypassing configuration is instead provided by a set of 3-terminal couplings 32 along the connection bus. Each 3-terminal coupling is a physical configuration rather than an electrical configuration, i.e. the wires which are for other ECG electrode connectors are not broken. Rather, one wire is physically tapped off from the connection bus to form the spur 30. Each spur is preferably a single wire. This means the ECG electrode connector is easy and light to manipulate.

However, the bus may instead have the same design along its length and the spurs may electrically connect to an associated wire in the bus.

The order of the ECG electrode connectors along the connection bus is again selected to achieve a desired cable routing when applied to the patient. As shown, the order is top right (of the patient's chest) =RA, top left=LA, bottom left=LL, center, bottom right=RL. Thus, the order along the bus is different to that shown in Figure 1, namely 18a, 18b, 18d, 18c, 18e.

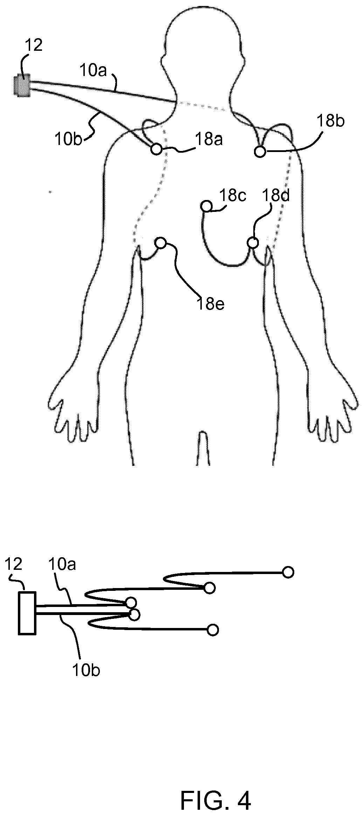

Figure 4 shows a third example in accordance with the invention. It again shows a 5-lead leadset with 5 ECG electrode connectors 18a to 18e. However, the connection bus is formed as a first connection bus 10a to which a first set of plurality of ECG electrode connectors connects, and a second connection bus 10b to which a second set of one or more ECG electrode connectors connects. The electrical terminal 12 is shared by the first and second connection buses 10a, 10b.

In this way, there are two connection buses extending from the terminal. This may make the routing to the full set of patient electrodes easier, for example using one bus for one side of the body and the other bus for the other side of the body.

In this example there are again 5 ECG electrode connectors, three 18b, 18d, 18c along the first connection bus 10a and two 18a and 18e along the second connection bus 10b. This provides a 2-bus 5 lead leadset.

Figure 4 is based on forked arrangements for the two connection buses.

The first connection bus 10a has electrode connectors LA, LL and center and the second connection bus has electrode connectors and RA and RL.

The 2-bus leadset keeps the torso more free of leads near the RL ECG electrode connector, because it is the last electrode connector along the second bus 10b. There are still only two buses, so that the likelihood of tangling is still reduced compared to a standard design.

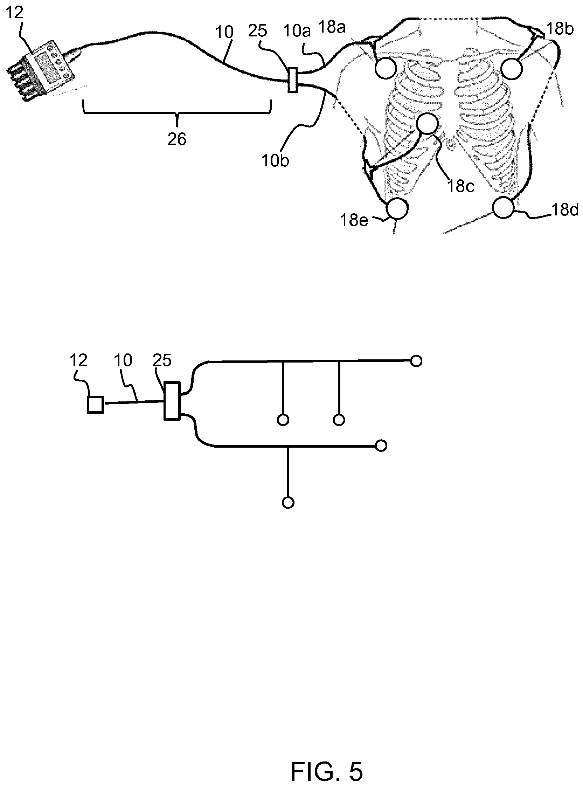

Figure 5 shows a fourth example in accordance with the invention. It again shows a 5-lead leadset with 5 ECG electrode connectors 18a to 18e and with a first connection bus 10a to which a first set of plurality of ECG electrode connectors connects, and a second connection bus 10b to which a second set of one or more ECG electrode connectors connects. The electrical terminal 12 is shared by the first and second connection buses 10a, 10b.

Furthermore, the two connection buses 10a, 10b are coupled together before the terminal 12 by a coupling piece 25. The coupling piece is thus between the first and second connection buses and the shared electrical terminal 12, and a combined bus 10 extends in region 26 between the coupling piece 25 and the shared electrical terminal 12. Thus, the two buses may be kept separate only for a required distance. They are coupled together before they reach the shared electrical terminal. This may be applied to all versions below which have multiple connection buses.

In this way, there are two connection buses extending from the terminal. This may make the routing to the full set of patient electrodes easier, for example using one bus for one side of the body and the other bus for the other side of the body.

In this example there are again 5 ECG electrode connectors, three 18a, 18b, 18d along the first connection bus 10a and two 18c and 18e along the second connection bus 10b. This provides a 2-bus 5 lead leadset. Figure 5 is based on spur arrangements for the two connection buses.

In this example the first connection bus 10a has electrode connectors RA, LA and LL and the second connection bus 10b has electrode connectors center and RL.

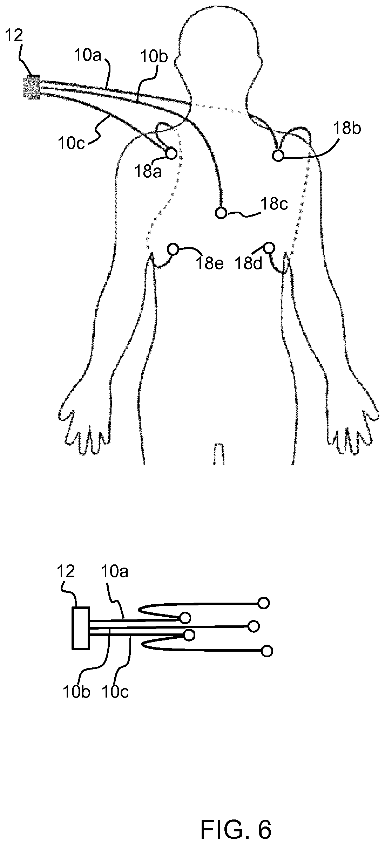

Figure 6 shows a fifth example in accordance with the invention. It again shows a 5-lead leadset with 5 ECG electrode connectors 18a to 18e but with three connection buses 10a to 10c. The electrical terminal 12 is shared by the first to third connection buses 10a to 10c.

The use of three connection buses extending from the terminal may make the routing to the full set of patient electrodes even easier.

In this example there are again 5 ECG electrode connectors, two 18b and 18d along the first connection bus 10a, one 18c along the second connection bus 10b and two 18a and 18e along the third connection bus. This provides a 3-bus 5 lead leadset. Figure 6 is based on forked arrangements for the three connection buses.

The 3 buses introduce symmetry in the leadset which makes it very simple to connect the leadset to the patient, even for persons with little training. The routing of the lead to the center ECG electrode connector 18c can be placed from the top (as shown) or from any other direction, whichever works best during surgery.

In this example, the first connection bus 10a has electrode connectors LA, LL, the second connection bus 10b has the center electrode connector and the third connection bus 10c has electrode connectors RA and RL.

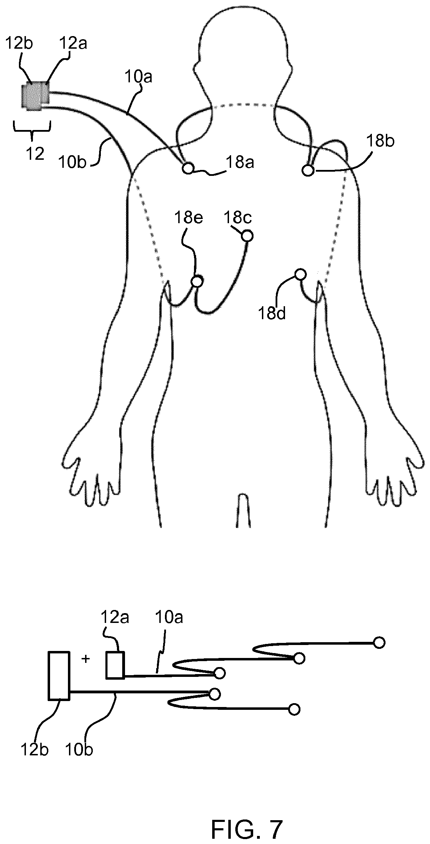

Figure 7 shows a sixth example in accordance with the invention. It shows a modification to the design of Figure 4, in which each of the two connection buses 10a, 10b each have their own end connector 12a, 12b, and the first and second end connectors are releasably coupled together to form the shared electrical terminal 12.

In this way, the leadset supports leadset extension, i.e. it may be used with a first number of ECG electrode connectors (3 in this example), or with a greater second number of ECG electrode connectors (5 in this example) by coupling together multiple buses (2 in this example).

In many clinical settings, an ECG is initially measured using 3 electrodes and a 3-lead leadset. During clinical investigations the need for 5-lead ECG measurement can occur. The three already placed patient electrodes and leadset can then remain in place, while two new ECG patient electrode contacts are placed and connected via a 2-lead extension leadset. The result in this example is a 5-lead measurement using a 2-bus leadset obtained from a 3-lead bus combined with a 2-lead extension bus. This arrangement is based on a forked arrangement but of course the same concept can be applied to a spur arrangement.

The end connector 12b functions as a feedthrough connector for the 3 electrode signals from the end connector 12a of the 3-lead bus 10a.

The general concept of leadset extension can of course be applied in several situations. Practical examples of clinical value are:
Extension from 3 lead to 5 lead;
Extension from 3 lead to 12 lead (10 electrodes);
Extension from 5 lead to 12 lead (10 electrodes).

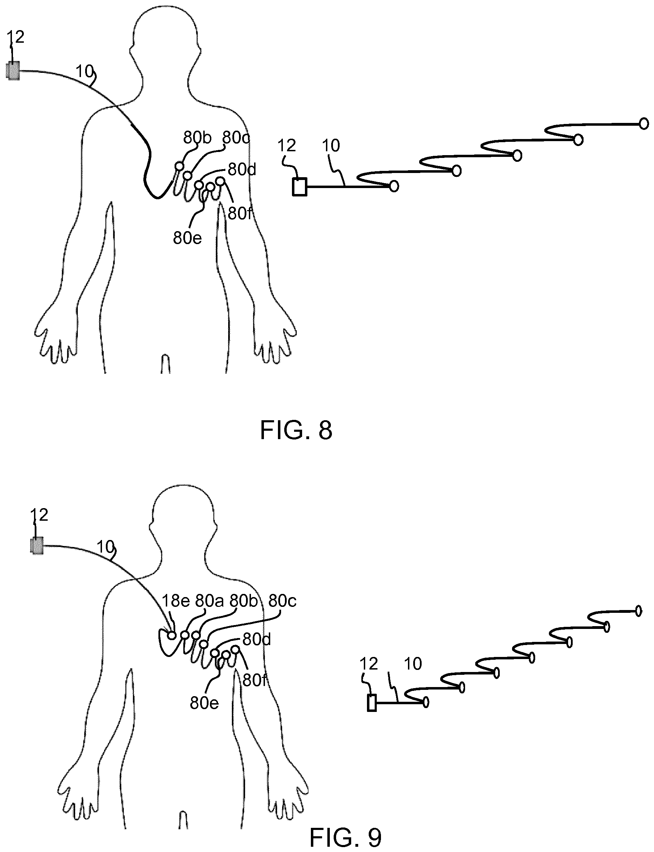

Figure 8 shows a seventh example in accordance with the invention. It shows a 5 electrode chest leadset connecting to the chest lead electrodes V2 to V6 by connectors 80b to 80f. It is based on a single connection bus architecture with a forked design. Since the locations for the chest lead electrodes V2 to V6 are naturally very close to each other in a well-defined pattern and order, a chest leadset based on a shared bus is of interest.

This 5 electrode leadset may be used to extend from a basic 5 lead ECG to 12 leads (10 ECG connections in total). Lead 18c from the original basic 5 lead ECG is used as the V1 chest lead electrode.

Figure 9 shows an eighth example in accordance with the invention. It shows a 7 electrode chest leadset connecting to the chest lead electrodes V1 to V6 by connectors 80a to 80f and additionally with connection 18e (RL). It is again based on a single connection bus architecture with a forked design.

This 7 electrode leadset may be used to extend from a basic 3 lead ECG to 12 leads (10 ECG connections in total).

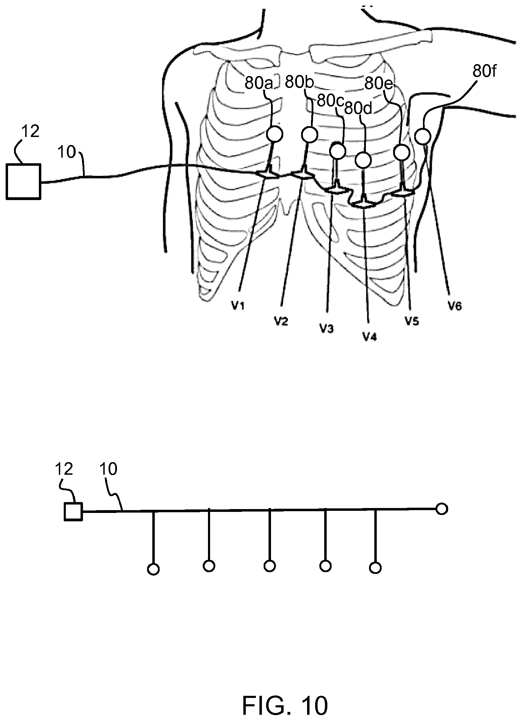

Figure 10 shows an ninth example in accordance with the invention. It shows a 6-electrode chest leadset connecting to the chest lead electrodes V1 to V6 but using a spur arrangement. The connectors are named 80a to 80f.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". Any reference signs in the claims should not be construed as limiting the scope.

## Claims

1. An ECG leadset, comprising:
a connection bus (10);
an electrical terminal (12) at one end of the connection bus; and
a set of a plurality of ECG electrode connectors (18a-18e; 80a-80f), each electrically connected to the connection bus at a different location along the bus, wherein the connection bus has at least one wire for each ECG electrode connector, and the electrical terminal (12) has at least one connection terminal for each wire,
wherein each ECG electrode connector has a single connection region (24) around the periphery of the ECG electrode connector at which it connects to the connection bus.

2. The ECG leadset as claimed in claim 1, wherein each ECG electrode connector of the plurality of electrode connectors, other than a last ECG electrode connector along the connection bus, connects to the connection bus through a spur connector (30) which extends between the connection bus and the connection region.

3. The ECG leadset as claimed in claim 2, wherein each spur connector (30) extends between the ECG electrode connector and a 3-terminal coupling (32) along the connection bus.

4. The ECG leadset as claimed in claim 2 or 3, wherein each spur connector (30) comprises a single wire.

5. The ECG leadset as claimed in claim 1, wherein each ECG electrode connector of the plurality of electrodes connectors connects to the connection bus (10) with a connection bus input (20) and a connection bus output (22), both at the connection region (24).

6. The ECG leadset as claimed in any one of claims 1 to 5 comprising exactly 3, 4, 5 or 6 ECG electrode connectors along the connection bus.

7. The ECG leadset as claimed in any one of claims 1 to 5, wherein the connection bus is a first connection bus (10a) to which the first set of plurality of ECG electrode connectors connects, and the ECG leadset further comprises a second connection bus (10b) to which a second set of one or more ECG electrode connectors connects, wherein the electrical terminal is shared by the first and second connection buses.

8. The ECG leadset as claimed in claim 7 comprising exactly 5 ECG electrode connectors, three belonging to the first set of ECG electrode connectors along the first connection bus (10a) and two belonging to the second set along the second connection bus (10b).

9. The ECG leadset as claimed in claim 7 or 8, comprising a coupling piece (25) between the first and second connection buses and the shared electrical terminal (12), with a combined bus (26) extending between the coupling piece and the shared electrical terminal.

10. The ECG leadset as claimed in any one of claims 7 to 9, wherein the first connection bus (10a) has a first end connector (12a) and the second connection bus (10b) has a second end connector (12b), the first and second end connectors being releasably couplable together to form the shared electrical terminal (12).

11. The ECG leadset as claimed in claim 7, comprising a third connection bus (10c) to which a third set of one or more ECG electrode connectors connects, wherein the electrical terminal (12) is shared by the first to third connection buses.

12. The ECG leadset as claimed in claim 11, comprising exactly 5 ECG electrode connectors, two along the first connection bus, two along the second connection bus and one along the third connection bus.

13. The ECG leadset as claimed in any one of claims 8 to 12, wherein each ECG electrode connector of each set, other than a last ECG electrode connector along the respective connection bus, connects to the respective connection bus through a spur connector.

14. The ECG leadset as claimed in claim 13, wherein each spur connector extends between the ECG electrode connector and a 3-terminal coupling along the respective connection bus.

15. The ECG leadset as claimed in any one of claims 8 to 12, wherein each ECG electrode connector of each set connects to the respective connection bus with a connection bus input and a connection bus output, both at the connection region.
